# EUROPEAN PATENT APPLICATION

(11) **EP 4 691 541 A1**
(43) Date of publication of application: **11.02.2026**
(21) Application number: 24199021.7
(22) Date of filing: 06.09.2024
(51) Int. Cl.: A61M 60/13, A61M 60/274, A61M 60/295, A61M 60/31, A61M 60/427, A61M 60/497, A61M 60/531, A61M 60/843

(54) **MULTI-BALLOON OUTSIDE AORTIC COUNTERPULSATION DEVICE**

(30) Priority: 05.08.2024 CN 202411065512
(71) Applicant: Taizhou MYDsn Medical Technology Co., Ltd, Taizhou Jiangsu 225316 (CN)
(72) Inventor: TAN, Xuerui, Beijing, 100043 (CN); JIA, Haitao, Tianjin, 301700 (CN); HE, Guojun, Wuhan, 430000 (CN)
(74) Representative: Murgitroyd & Company

(57) **Abstract**

The present invention discloses a multi-balloon outside aortic counterpulsation device, including an in vivo catheter, a balloon cavity, and IABP balloons disposed in the balloon cavity, wherein an end of each of the IABP balloons extends out of the balloon cavity for connection with a controller. A side wall of an inner end of the in vivo catheter is provided with several through holes, and an outer end of the in vivo catheter is in airtight connection with a first interface of the balloon cavity; and a plurality of the IABP balloons arranged in parallel are included in the balloon cavity, each of the IABP balloons extends out of the balloon cavity through a seal valve, and the plurality of IABP balloons implements inflation and deflation actions at the same time, wherein when the plurality of IABP balloons implements deflation at the same time, blood is drawn into the balloon cavity, and when the plurality of IABP balloons implements inflation at the same time, the blood inside the balloon cavity is pushed back into a body, allowing for the flow of blood. According to the present invention, the inflation and deflation actions of the external balloons allow for extrusion and drawing of the blood inside the balloon cavity, and the in vitro balloon cavity can accommodate more balloons to achieve the technical effect of doubling the cardiac output, thereby meeting the patient need for systemic blood supply.

## Description

### BACKGROUND OF THE INVENTION

### 1. Technical Field

The present invention relates to the technical field of medical devices, and in particular to a multi-balloon outside aortic counterpulsation device.

### 2. Description of Related Art

An intra-aortic balloon pump, a mechanical device designed according to the principle of counterpulsation to assist the failing left heart, is typically used for in-hospital rehabilitation after interventional heart surgery, heart attack or other major cardiac events. It is the most commonly used auxiliary device in the perioperative period of PCI, particularly suitable for providing advanced life support for critically ill cardiac patients, and can effectively improve the coronary blood supply as well as low cardiac output and hypotension for the patients. The basic principle of IABP is as follows: a balloon is disposed in the descending aorta to inflate during diastole and deflate during systole, achieving the effect of assisting cardiac circulation. Inflating the intra-aortic balloon during diastole can increase the diastolic blood pressure and thus the blood flow in the coronary artery to improve myocardial blood and oxygen supply while increasing the blood perfusion to the brain, kidneys and peripheries; and quickly retracting and emptying the balloon during systole can induce "cavitation" to reduce the left ventricular afterload and thus reduce myocardial oxygen consumption.

In the interventional therapies for cardiovascular diseases, IABP is the most commonly used auxiliary device in the perioperative period of PCI. According to the data from the National Cardiovascular Data Registry of the United States from 2009 to 2013, 89.3% of patients supported by pMCS used IABPs, and only 10.7% used other auxiliary devices. At present, all the IABPs in China rely on imports. Major manufacturers across the world involves three brands, including Getinge Group, Teleflex, and Senko Medical Instrument Mfg. Co., Ltd (MERA). In 2020, the share of Getinge accounted for more than 80%. As for the selection criteria for IABPs, the size of a balloon is determined depending on height. At present, Teleflex's Arrow IABP system has the catheter available with the diameter of 7 fr, 7.5 fr and 8fr, and the balloon available with the capacity of 30 cc, 40 cc and 50cc.

From the comparison of IABP, ECMO, and artificial heart in usage, IABP shows absolute advantages in sheath size, bedside placement, operation difficulty, indwelling time, postoperative management requirements, hemolysis risk or the like, with the sole defect of excessively low cardiac output, which is 0.5-1 L per minute. Consequently, it is significantly inferior to ECMO and the artificial heart in the actual use effect on the patients. In view of this major defect of the IABP device, international and national physicians and engineers have tried many methods, but with little effect. As a result, there is no effective method to solve the problem of excessively low cardiac output of the IABP device.

On this basis, the application makes full use of the advantages of the IABP device to creatively propose a multi-balloon outside aortic counterpulsation device, in which balloons disposed in vitro in an outside aortic counterpulsation mode are enabled to maximize the cardiac output and thus solve the problem of excessively low cardiac output of the IABP device.

### BRIEF SUMMARY OF THE INVENTION

The technical problem to be solved by the present invention is to provide a multi-balloon outside aortic counterpulsation device, which is enabled to maximize the cardiac output and solve the problem of excessively low cardiac output of an IABP device.

To solve the technical problem above, the present invention provides a multi-balloon outside aortic counterpulsation device, including an in vivo catheter for extending into a descending aorta, a balloon cavity connected to an in vitro end of the in vivo catheter, and IABP balloons disposed in the balloon cavity, wherein an end of each of the IABP balloons extends out of the balloon cavity for connection with a controller that controls implementation of inflation and deflation of the balloons;
a side wall of an inner end of the in vivo catheter is provided with several through holes, and an outer end of the in vivo catheter is in airtight connection with a first interface of the balloon cavity, to achieve communication between the in vivo catheter and an interior of the balloon cavity; and
a plurality of the IABP balloons arranged in parallel are comprised in the balloon cavity, each of the IABP balloons extends out of the balloon cavity through a seal valve, and the plurality of IABP balloons implements inflation and deflation actions at the same time, wherein when the plurality of IABP balloons implements deflation at the same time, a space of the balloon cavity occupied by the balloons decreases to draw blood from the descending aorta into the balloon cavity, and when the plurality of IABP balloons implements inflation at the same time, the space of the balloon cavity occupied by the balloons increases to push the blood inside the balloon cavity back into a body, achieving an outside aortic blood counterpulsation effect.

As a further improvement, the balloon cavity includes 2-10 IABP balloons.

As a further improvement, the first interface of the balloon cavity and an outer end interface of the in vivo catheter are provided with Luer fittings matching each other.

As a further improvement, a pressure sensor is disposed on an outer side of a tip of an inner end of the in vivo catheter.

As a further improvement, the in vivo catheter has a lumen diameter of 2.2-9 mm, a perforated section of the in vivo catheter has a length of 320-520 mm, each of the through holes has a diameter of 1-4 mm, and a spacing between every two adjacent through holes is 1-10 mm.

As a further improvement, a diaphragm for dividing an interior of the catheter into a blood drawing cavity and a perfusion cavity is further disposed inside the in vivo catheter, one end of the diaphragm is located at a beginning of the perforated section of the in vivo catheter, and the other end of the diaphragm is located at a tip of the in vitro end of the in vivo catheter.

As a further improvement, both sides of the diaphragm are adhered to an inner side wall of the in vivo catheter, and both ends of the diaphragm are inclined such that an inner end of the blood drawing cavity and an outer end of the perfusion cavity are each of a flared structure.

As a further improvement, the diaphragm is made of an elastic thin film.

As a further improvement, the in vivo catheter is made of a polymer material such as PTFE, PU, Pebax or PE and prepared by a blow molding or extrusion process, the balloon cavity is made of silicone rubber, polyurethane, PTFE, PU, Pebax, PE, or other polymer materials having a hardness of more than 65 and prepared by an injection molding or blow molding process, and the thin film is made of silicone rubber, polyurethane, PTFE, PU, Pebax, PE, or other elastic medical polymer materials having a hardness of less than 30.

As a further improvement, the controller is an IABP system controller to supply an inflation and deflation gas to the IABP balloons.

In such a design, the present invention has at least the following advantages.
1. The multi-balloon outside aortic counterpulsation device of the present invention extrudes and draws the blood inside the balloon cavity by external arrangement of the IABP balloons that implement the inflation and deflation actions in the balloon cavity in vitro, thereby achieving external counterpulsation of the blood in the descending aorta. The multi-balloon outside aortic counterpulsation device can accommodate more balloons by means of the balloon cavity in vitro, and the plurality of balloons acting at the same time can achieve the technical effect of doubling the cardiac output, which truly overcomes the problem of low cardiac output caused by the fact that the inflation and deflation actions of only one balloon are available in the case of in vivo arrangement of the balloon. Moreover, the device can be completely based on the existing IABP device and IABP balloon, showing 100% compatibility, low cost and good effect and allowing for the cardiac output of more than 5 L/min, which meets the patient need for systemic blood supply and greatly improves the blood supply status of the patient. In addition, the device allows the balloons to keep away from the aortic blood vessels, such that blockage can be performed in time even in the case of a high-risk event such as the gas leakage of the balloon, thereby preventing adverse effects on the patient.
2. Furthermore, the diaphragm is disposed in the in vivo catheter to form a dual-channel structure in the in vivo catheter, allowing for blood drawing at one side and blood perfusion at the other side, such that the two channels do not affect each other to avoid problems such as blood collision and serious turbulence in the case of a single channel.
3. Furthermore, the elastic thin film is disposed with both ends provided with flared structures, which can effectively guarantee the unidirectional flow of blood and allow for maximum unidirectional cross section of the blood during drawing and perfusion, thereby greatly reducing the external power for better implementation of the unidirectional flow of blood.

### BRIEF DESCRIPTION OF THE SEVERAL VIEWS OF THE DRAWINGS

Described above is only an outline of the technical solution of the present invention. For clearer understanding of the technical means of the present invention, the present invention will be further illustrated in detail below in conjunction with the accompanying drawings and the specific embodiments.
Fig. 1 is a schematic structural diagram of a multi-balloon outside aortic counterpulsation device applied to a human body, according to the present invention;
Fig. 2 is a schematic structural diagram of an in vivo catheter in the multi-balloon outside aortic counterpulsation device according to the present invention;
Fig. 3 is a schematic structural diagram of a balloon cavity, with IABP balloons inflated, in the multi-balloon outside aortic counterpulsation device according to the present invention;
Fig. 4 is a schematic structural diagram of the balloon cavity, with the IABP balloons deflated, in the multi-balloon outside aortic counterpulsation device according to the present invention;
Fig. 5 is a schematic structural diagram of an end of the balloon cavity provided with a hemostatic valve, in the multi-balloon outside aortic counterpulsation device according to the present invention;
Fig. 6 is a schematic structural diagram of a diaphragm section of the in vivo catheter in the outside aortic counterpulsation device according to the present invention;
Fig. 7 is a schematic section view of the diaphragm section of the in vivo catheter in the outside aortic counterpulsation device according to the present invention (with a blood drawing cavity expanded); and
Fig. 8 is a schematic section view of the diaphragm section of the in vivo catheter in the outside aortic counterpulsation device according to the present invention (with a perfusion cavity expanded).

### DETAILED DESCRIPTION OF THE INVENTION

The exemplary embodiments of the present invention will be described below in more details with reference to the accompanying drawings. Although the exemplary embodiments of the present invention are shown in the accompanying drawings, it should be understood that the present invention can be implemented in various forms and should not be limited by the embodiments set forth herein. On the contrary, these embodiments are provided to enable a more thorough understanding of the present invention and to fully convey the scope of the present invention to those skilled in the art.

It should also be noted that the terms such as "left", "right", "inside", and "outside" in the present invention indicate directions or positional relations based on the directions or positional relations as shown in the accompanying drawings only for the convenience of describing the present invention and simplifying the description, instead of indicating or implying that a mentioned device or element must have a specific direction or must be constructed and operated in a specific direction. Therefore, these terms should not be construed as limiting the present invention.

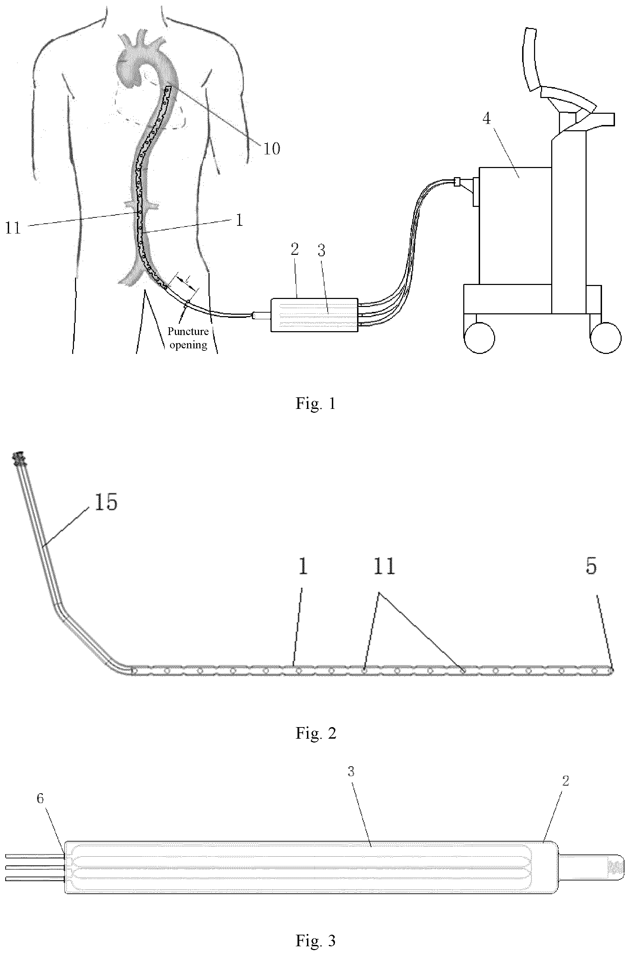

Referring to Fig. 1, a multi-balloon outside aortic counterpulsation device in this embodiment includes an in vivo catheter 1 for extending into a descending aorta, a balloon cavity 2 connected to an in vitro end of the in vivo catheter 1, and IABP balloons 3 disposed in the balloon cavity 2. An end of each of the IABP balloons 3 extends out of the balloon cavity 2 for connection with a controller 4 that controls implementation of inflation and deflation actions of the balloons.

Referring to Fig. 2, a side wall of an inner end of the in vivo catheter 1 is provided with several through holes 11, an outer end of the in vivo catheter 1 is provided as a Luer fitting, and the first interface of the balloon cavity 2 connected to the in vivo catheter 1 is also provided as a Luer fitting for airtight connection between the in vivo catheter 1 and the balloon cavity 2, achieving communication between the in vivo catheter 1 and the interior of the balloon cavity 2. That is, when the in vivo catheter 1 enters the body, the in vivo catheter 1 and the balloon cavity 2, together with the blood vessels in the body, form a closed space, and blood flows in pathways formed in the closed space. This space is an extension section of the aortic blood vessel of the human body. Of course, any other existing airtight connection mechanism may also be used for the in vivo catheter 1 and the balloon cavity 2, as long as the airtight connection between the in vivo catheter 1 and the balloon cavity 2 is guaranteed.

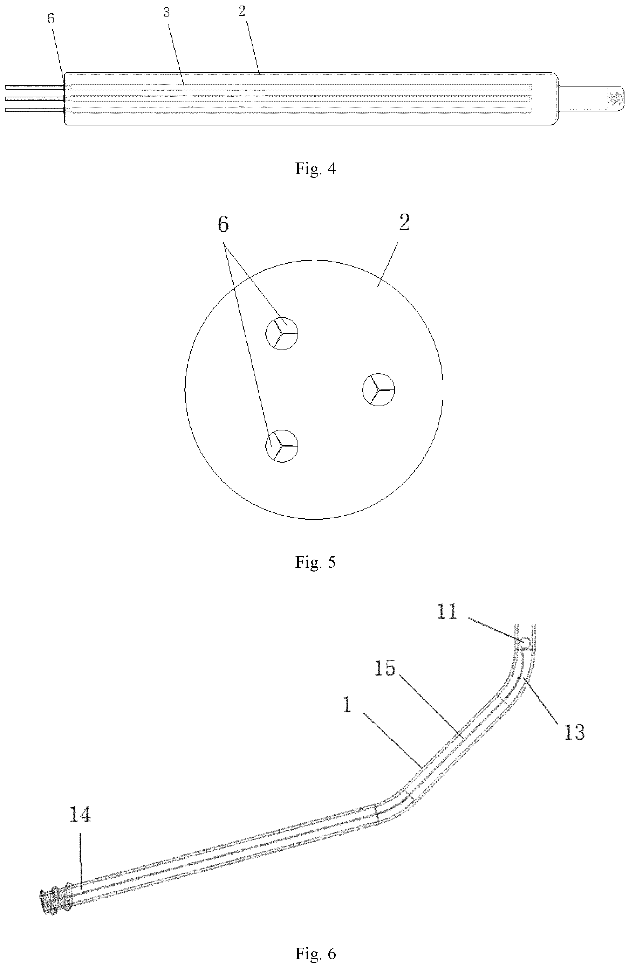

Referring to Figs 3 to 5, the balloon cavity 2 includes a plurality of IABP balloons 3 arranged in parallel, and each of the IABP balloons 3 extends out of the balloon cavity 2 through a seal valve 6, preferably a hemostatic valve, to achieve the airtight connection between the IABP balloon 3 and the balloon cavity 2 and thus prevent the blood from flowing out, as shown in Fig. 5. In this embodiment, an existing IABP balloon may be used as the IABP balloon 3, with a head end provided with an inflation balloon, for which a gas comes from the controller 4 and which can implement the inflation and deflation actions.

It should be noted that the plurality of IABP balloons 3 in this embodiment needs to implement the inflation and deflation actions at the same time; when the plurality of IABP balloons 3 implements deflation at the same time, the space of the balloon cavity 2 occupied by the balloons decreases to draw blood from the descending aorta 10 into the balloon cavity 2 by means of "cavitation", as shown in Fig. 4; and when the plurality of IABP balloons 3 implements inflation at the same time, the space of the balloon cavity 2 occupied by the balloons increases to push the blood inside the balloon cavity 2 back into a body, as shown in Fig. 3, thereby achieving an outside aortic blood counterpulsation effect.

In this embodiment, the balloon cavity 2 includes 2-10 IABP balloons 3, preferably 3-6 IABP balloons, such that both reduced losses and the cardiac output of greater than 5 L/min can be achieved. To accommodate the IABP balloons 3, the total length of the balloon cavity 2 is greater than that of a balloon section of the IABP balloons, and is in a range of 200-400 mm.

Specifically, the overall length of the in vivo catheter 1 is 500-1000 mm, preferably 600 mm, 750 mm or 900 mm, so as to adapt to the human body of a different height. The outer diameter of the in vivo catheter 1 is at least 7 F, and at most 24 F. That is, the in vivo catheter has a lumen diameter of 2.2-9 mm. The principle for setting the diameter of the in vivo catheter is that: the inner lumen diameter of the aortic blood vessel in a human body is generally about 10 mm, the outer diameter of the catheter must be smaller than the inner diameter of the aortic blood vessel, with a certain gap reserved, and the gap of 0.5 mm at one side is necessary, therefore, the outer diameter of the catheter cannot exceed 9 mm at most, otherwise the inner wall of the aortic blood vessel will be damaged. Of course, the catheter should not be too thin, and if so, it is laborious to draw and perfuse the blood and the load is very large; moreover, liquid hysteresis becomes very significant; and meanwhile, vacuum may occur during vigorous drawing, which will damage blood cells or induce aeroembolism to endanger the life safety of patients.

Also, a perforated section of the in vivo catheter 1 provided with the through holes 11 has a length of 320-520 mm. A plurality of the through holes 11 are provided for reducing the load during blood drawing and perfusion, allowing for the flow of blood easily. The through holes 11 each have a diameter of 1-4 mm and are evenly distributed around the wall of the in vivo catheter in a mode that is not limited. The through holes may be distributed spirally, equidistantly in circles, or optimally in such a way to ensure an equal distance between any two through holes, with the distance in a range of 1-10 mm. In case of a too small distance, the overall rigidity of the catheter may be destroyed, and in case of a too large distance, the drawing load may be increased. The same is true for the aperture, where in case of a too small aperture, the drawing load will be increased, leading to increased difficulty, and in case of a too large aperture, the overall rigidity will be destroyed, making the catheter easy to damage. Notably, the initial position for perforation in the wall of the catheter should not be too close to a puncture opening for accessing to the body, otherwise, blood oozing may be caused easily. The optimal range of L from an initial perforation section to the position of a puncture point is: 20-80 mm. In case of a too short distance, blood oozing may be caused due to high pressure during blood drawing and perfusion, and in case of a too long distance, a blind cavity region unperforated will become large, leading to increased drawing load and thus increased difficulty in blood drawing. The optimal range of L is 30-50 mm.

In this embodiment, a pressure sensor 5 is disposed on the outer side of a tip of an inner end of the in vivo catheter 1 for monitoring the blood pressure condition in real time and transmitting signals to the controller 4 to create a control trigger mode.

The application principle of the outside aortic counterpulsation device is that: the in vivo catheter 1 is disposed in the aorta through the puncture surgery, and the end of the in vivo catheter is located in the descending aorta 10 and reaches at most, without entering, the aortic arch, preferably at a distance of about 100 mm from the bend of the aortic arch. The surgical puncture may be performed in the femoral artery, the radial artery, or the carotid artery. After the puncture is completed for the in vivo catheter 1, the blood in vivo is directed along the catheter into the balloon cavity 2. The inflation and deflation of the IABP balloons 3 are controlled by means of the controller 4 to direct the blood from the descending aorta 10 in vivo to the in vitro balloon cavity 2 along the catheter via the through holes 11, and then perfuse the blood back to the descending aorta 10 in vivo, achieving outside aortic blood counterpulsation.

In a preferred embodiment, as shown in Fig. 6, a diaphragm 15 for dividing an interior of the catheter into a blood drawing cavity 13 and a perfusion cavity 14 is further disposed inside the in vivo catheter 1, one end of the diaphragm 15 is located at a beginning of the perforated section of the in vivo catheter 1, and the other end of the diaphragm 15 is located at a tip of the in vitro end of the in vivo catheter 1.

Specifically, both sides of the diaphragm 15 are adhered to an inner side wall of the in vivo catheter 1 respectively, and both ends of the diaphragm 15 are inclined such that an inner end of the blood drawing cavity 13 and an outer end of the perfusion cavity 14 are each of a flared structure, as shown in Fig. 6, for guiding the flow of blood in the in vivo catheter, to ensure the smooth access of the blood.

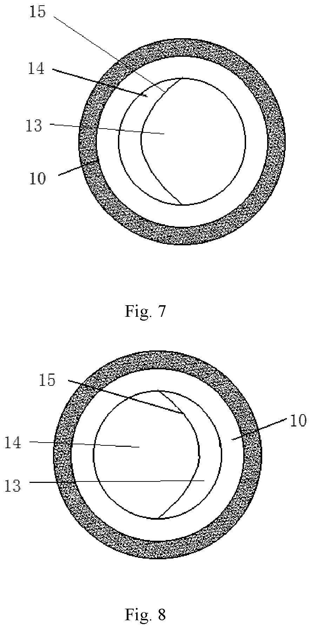

More preferably, the diaphragm 15 is an elastic thin film. The thin film is made of an elastic medical polymer material such as silicone rubber, polyurethane, PTFE, PU, Pebax or PE. The optimal hardness range of the elastic material is less than 30. During blood drawing, the elastic thin film deforms to the left, i.e. extruding the perfusion cavity 14, such that the blood drawing cavity 13 increases and the perfusion cavity 14 decreases, as shown in Fig. 7. During perfusion, the thin film deforms to the right, i.e., extruding the blood drawing cavity 13, such that the perfusion cavity 14 increases and the blood drawing cavity 13 decreases, as shown in Fig. 8. As a result, the adaptive adjustment is achieved during drawing and perfusion. In an initial state, the thin film is centralized to equally divide the lumen of the catheter. That is, the blood drawing cavity 13 and the perfusion cavity 14 have the same cross section area. The reason for designing the dual-cavity structure lies in that: in case of a single-channel catheter, the blood in the lumen always repeats the process of acceleration-deceleration-stop-reverse-acceleration →revers→deceleration→stop during blood drawing and perfusion, and in this process, the liquid cannot be fed back quickly due to high frequency and fast speed, naturally leading to vacuum and thus conditions such as mutual collision and serious turbulence; and in case of dual channels provided, the blood may be drawn from one end and perfused back from the other end, without mutual influence, which may increase the corresponding speed and eliminate vacuum and turbulence and consequently greatly reduce the required external power. Therefore, the catheter of the dual-cavity structure of the present invention can ensure the unidirectional flow of the blood. Furthermore, in case of one inelastic thin film provided to divide a lumen into two cavities, the cross section of a single cavity becomes very small and is less than or equal to half of the original cross section. In this case, the difficulty in blood drawing and perfusion increases. Therefore, the creatively disposed elastic thin film with both ends provided with flared structures according to the present invention can effectively guarantee the unidirectional flow of blood and allow for maximum unidirectional cross section of the blood in respective process, thereby greatly reducing the external power for better implementation of the unidirectional flow of blood.

More specifically, the in vivo catheter 1 is made of a polymer material such as PTFE, PU, Pebax or PE and prepared by a blow molding or extrusion process; and the balloon cavity 2 is made of silicone rubber, polyurethane, PTFE, PU, Pebax, PE or other materials having the hardness of more than 65 and molded by a process such as injection molding or blow molding.

In this embodiment, the controller 4 is an existing IABP system controller for supplying a gas to the IABP balloons 3. Preferably, a helium pump is disposed in the controller 4 to supply helium to the IABP balloons.

The controller 4 has the following functions: 1) waveform display: ECG, Ap and BP waveforms, ECG adjustable piston action interval, and accurate display of catheter pressure; 2) physiological data display: heart rate, assisted systolic/diastolic/mean/counterpulsation blood pressure, unassisted systolic/diastolic/mean blood pressure; 3) icon display: battery capacity, piston status, and accurate display of the pressure value in the respiratory cavity; 4) control mode: single touch screen control, button control, alarm angle control, and dual control of key/common functions: touch screen/buttons for auxiliary start, auxiliary frequency, screen freeze, print, and guide line setting; 5) operating mode: including automatic and manual modes, which can be switched without affecting the normal counterpulsation and with the original settings reserved automatically, where the automatic mode is available for automatic selection of signal source, trigger mode (6 types), time phase algorithm and optimal ECG lead (7 types), and real-time evaluation of ECG guide status, and the manual mode is available for selection of signal source, trigger mode, and ECG lead, and adjustment of time phase; 6) trigger mode: 7 types, including pattern mode, peak mode, Aifb mode, pacemaker V/A-V mode, pacemaker A mode, AP mode, and inboard setting mode; 7) balloon delay analysis: real-time calculation of balloon perfusion speed and evaluation of R-wave deflation safety; 8) auxiliary frequency: 4 types, including 1:1/1:2/1:4/1:8; 9) counterpulsation frequency: up to 200 times/min; 10) counterpulsation capacity: 0-150 ml each time, with the adjustable accuracy of 0.5 ml; 11) automatic water removal: automatic removal of condensed water (produced during cooling of a driver that produces heat during working, and needing removal before the device is used) every 20 times, without affecting normal assistance; 12) patient data report: display and print of all the recorded patient information related to counterpulsation; 13) power-on self-test checklist: indicating function self-test results in a list; and 14) alarm history: display and print of the most recent 100 alarms.

Of course, the controller 4 may also have other structures, as long as similar functions of the IABP system controller can be achieved.

According to the multi-balloon outside aortic counterpulsation device of the present invention, the IABP balloons are disposed in vitro, instead of in vivo as the IABP system, since the length and diameter of the aortic blood vessel of a human body are limited in vivo, and the IABP device is available at most in three capacities, 30 cc, 40 cc, and 50 cc, and can thus supply the maximal cardiac output of 1 L/min at most; by placing the IABP balloons in vitro, their number and capacity can be designed according to the maximum cardiac output to allow the actual cardiac output to be greater than 5 L/min, which thoroughly overcomes the huge defect of insufficient cardiac output of the IABP; and furthermore, the overall device still retains all the advantages of the IABP, and shows high compatibility and low cost.

The above description only provides preferred embodiments of the present invention, and is not intended to limit the present invention in any form. Some simple alternations, equivalent variations or modifications made by those skilled in the art by using the technical content disclosed above shall fall within the protection scope of the present invention.

## Claims

1. A multi-balloon outside aortic counterpulsation device, comprising an in vivo catheter for extending into a descending aorta, a balloon cavity connected to an in vitro end of the in vivo catheter, and IABP balloons disposed in the balloon cavity, wherein an end of each of the IABP balloons extends out of the balloon cavity for connection with a controller that controls implementation of inflation and deflation of the balloons;
a side wall of an inner end of the in vivo catheter is provided with several through holes, and an outer end of the in vivo catheter is in airtight connection with a first interface of the balloon cavity, to achieve communication between the in vivo catheter and an interior of the balloon cavity; and
a plurality of the IABP balloons arranged in parallel are comprised in the balloon cavity, each of the IABP balloons extends out of the balloon cavity through a seal valve, and the plurality of IABP balloons implements inflation and deflation actions at the same time, wherein when the plurality of IABP balloons implements deflation at the same time, a space of the balloon cavity occupied by the balloons decreases to draw blood from the descending aorta into the balloon cavity, and when the plurality of IABP balloons implements inflation at the same time, the space of the balloon cavity occupied by the balloons increases to push the blood inside the balloon cavity back into a body, achieving an outside aortic blood counterpulsation effect.

2. The multi-balloon outside aortic counterpulsation device according to claim 1, wherein the balloon cavity comprises 2-10 IABP balloons.

3. The multi-balloon outside aortic counterpulsation device according to claim 1, wherein the first interface of the balloon cavity and an outer end interface of the in vivo catheter are provided with Luer fittings matching each other.

4. The multi-balloon outside aortic counterpulsation device according to claim 1, wherein a pressure sensor is disposed on an outer side of a tip of an inner end of the in vivo catheter.

5. The multi-balloon outside aortic counterpulsation device according to claim 1, wherein the in vivo catheter has a lumen diameter of 2.2-9 mm, a perforated section of the in vivo catheter has a length of 320-520 mm, each of the through holes has a diameter of 1-4 mm, and a spacing between every two adjacent through holes is 1-10 mm.

6. The multi-balloon outside aortic counterpulsation device according to any one of claims 1 to 5, wherein a diaphragm for dividing an interior of the catheter into a blood drawing cavity and a perfusion cavity is further disposed inside the in vivo catheter, one end of the diaphragm is located at a beginning of the perforated section of the in vivo catheter, and the other end of the diaphragm is located at a tip of the in vitro end of the in vivo catheter.

7. The multi-balloon outside aortic counterpulsation device according to claim 6, wherein both sides of the diaphragm are adhered to an inner side wall of the in vivo catheter respectively, and both ends of the diaphragm are inclined such that an inner end of the blood drawing cavity and an outer end of the perfusion cavity are each of a flared structure.

8. The multi-balloon outside aortic counterpulsation device according to claim 7, wherein the diaphragm is made of an elastic thin film.

9. The multi-balloon outside aortic counterpulsation device according to claim 8, wherein the in vivo catheter is made of a polymer material such as PTFE, PU, Pebax or PE and prepared by a blow molding or extrusion process, the balloon cavity is made of silicone rubber, polyurethane, PTFE, PU, Pebax, PE, or other polymer materials having a hardness of more than 65 and prepared by an injection molding or blow molding process, and the thin film is made of silicone rubber, polyurethane, PTFE, PU, Pebax, PE, or other eastic medical polymer materials having a hardness of less than 30.

10. The multi-balloon outside aortic counterpulsation device according to claim 1, wherein the controller is an IABP system controller to supply an inflation and deflation gas to the IABP balloons.
